(19) Europäisches Patentamt
European Patent Office
Office européen des brevets

(11) Numéro de publication : **0 068 998**
**B1**

(12) # FASCICULE DE BREVET EUROPÉEN

(45) Date de publication du fascicule du brevet : **28.08.85**

(21) Numéro de dépôt : **82401128.2**

(22) Date de dépôt : **21.06.82**

(51) Int. Cl.[4] : **C 07 C101/28**, C 07 D211/14, C 07 D295/14, C 07 D307/14, A 61 K 31/215, A 61 K 31/34, A 61 K 31/395

(54) **Aryl-1 aminométhyl-2 cyclopropanes carboxylates (Z), leur préparation et leur application en tant que médicaments utiles dans le traitement d'algies diverses.**

(30) Priorité : **23.06.81 FR 8112311**

(43) Date de publication de la demande : **05.01.83 Bulletin 83/01**

(45) Mention de la délivrance du brevet : **28.08.85 Bulletin 85/35**

(84) Etats contractants désignés : **AT BE CH DE GB IT LI LU NL SE**

(56) Documents cités :
**CHEMICAL ABSTRACTS, vol.90, no.11, 12 mars 1979, page 561, résumé no.86788f, Columbus, Ohio (US) S. CASADIO et al.: "1-Phenyl-2-(hydroxymethyl)cyclopropanecarboxylic acid and derivatives"**

(73) Titulaire : **PIERRE FABRE S.A.**
**125, rue de la Faisanderie**
**F-75116 Paris (FR)**

(72) Inventeur : **Cousse, Henri**
**La Foun de los Nobios Chemin de Lastinos**
**F-81100 Castres (FR)**
Inventeur : **Mouzin, Gilbert**
**21, rue Sainte-Foy**
**F-81100 Castres (FR)**
Inventeur : **Bonnaud, Bernard**
**2, rue Georges Bizet**
**F-81100 Castres (FR)**
Inventeur : **Charveron, Marie**
**18, avenue de Lameilhé**
**F-81100 Castres (FR)**
Inventeur : **Fauran, François**
**Bouisset-Larège**
**F-31230 Castanet Tolosan (FR)**

(74) Mandataire : **Corre, Jacques Denis Paul et al**
**Cabinet Regimbeau 26, Avenue Kléber**
**F-75116 Paris (FR)**

**EP 0 068 998 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention, réalisée au Centre de Recherches Pierre Fabre, concerne de nouveaux dérivés d'ester aryl-1 aminométhyl-2 cyclopropanes carboxyliques (Z), leur procédé de préparation et leur utilisation en thérapeutique et en particulier dans le traitement des algies diverses.

La technique antérieure la plus proche connue peut par exemple être illustrée par le brevet français n° 75 07 120 concernant un procédé de préparation d'acides aryl-1 hydroxyméthyl-2 cyclopropanes carboxyliques.

Ces dérivés acide-alcools ont par ailleurs fait l'objet d'une publication de G. Mouzin, H. Cousse et B. Bonnaud dans Synthesis 1978, 304, reprise dans Synthetic methods of organic chemistry (Ed. W. Theilheimer) 34, 1980, 317. L'étude pharmacologique, des dérivés de l'acide phényl-1 hydroxyméthyl-2 cyclopropane carboxylique décrits par S. Casadio, B. Bonnaud, G. Mouzin et H. Cousse dans Boll. Chim. Farm. 117, 1978, 331, a montré la faible activité pharmacologique de ces dérivés.

La présente invention se rapporte à de nouveaux composés, se distinguant de ceux de la technique antérieure précitée ; il s'agit de dérivés esters aminés cyclopropaniques, répondant à la formule

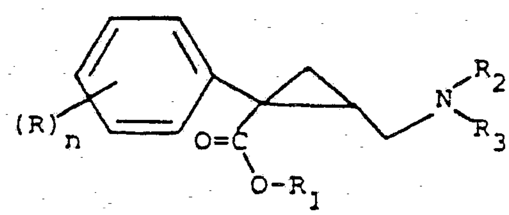

Or, il a été démontré que les modulations effectuées au niveau des groupes fonctionnels portés par le groupe cyclopropanique conféraient à ces nouveaux esters aminés cyclopropaniques de fort intéressantes propriétés pharmacologiques, et plus particulièrement une action antalgique qui permet d'utiliser ces composés dans le traitement d'algies diverses.

La présente invention concerne de nouveaux dérivés d'aryl-1 aminométhyl-2 cyclopropanes carboxylates (Z) de formule générale I

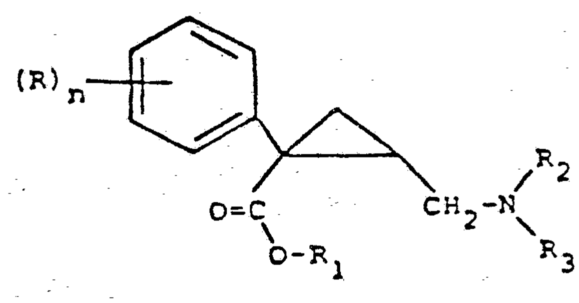

(I)

dans laquelle

R représente un atome d'halogène, un groupe alcoyle inférieur en $C_1$ à $C_4$, alcoxy inférieur en $C_1$ à $C_4$, nitro, amino, sulfamoyle ou hydroxy ;

n représente les valeurs 1, 2 ou 3 ;

$(R)_n$ peut également former avec le cycle benzénique le noyau naphtyle ;

$R_1$ représente un groupe alcoyle ou alcényle linéaires ou ramifiés en $C_1$ à $C_5$, aryle ou benzyle ;

$R_2$ et $R_3$ représentent un atome d'hydrogène, un groupe alcoyle, alcényle, alcynyle, hydroxyalcoyle, alcoxyalcoyle, carboxyalcoyle ou dialcoylaminoalcoyle linéaires ou ramifiés en $C_1$ à $C_5$, aryle, arylalcoyle ou cycloalcoyle ;

$R_2$ et $R_3$ pouvant également former avec l'atome d'azote voisin un hétérocycle à 5 ou 6 chaînons.

L'hétérocycle formé par les radicaux $R_2$ et $R_3$ avec l'atome d'azote voisin désigne plus particulièrement un hétérocycle de caractère non aromatique à 5 ou 6 chaînons pouvant éventuellement contenir un ou plusieurs autres hétéroatomes principalement choisis parmi l'azote et l'oxygène et pouvant en outre être substitué, par exemple en position para dudit atome d'azote voisin ou sur le second hétéroatome par un radical aryle, en particulier un radical phényle lui-même éventuellement substitué par un ou plusieurs autres atomes d'halogène, tel que le chlore.

La présente invention concerne également les sels des composés de formule générale I avec des acides minéraux ou organiques thérapeutiquement acceptables. A titre d'exemples non limitatifs de ces sels on mentionnera les halogénohydrates, tels que le chlorhydrate, le fumarate, le maléate, l'oxalate, le citrate et le glutamate.

La présente invention concerne également un procédé de préparation des composés de formule

générale (I) consistant à traiter la lactone de formule générale (II) par un alcool ou un phénol en présence d'un halogénure de thionyle. La lactone de formule générale (II) peut par exemple être obtenue par le procédé de préparation décrit dans le brevet français n° 75 07 120 appartenant à la demanderesse. L'intermédiaire halogéné de formule générale (III) est ensuite condensé sur une amine. Le procédé de préparation est illustré par le schéma réactionnel suivant.

Schéma réactionnel

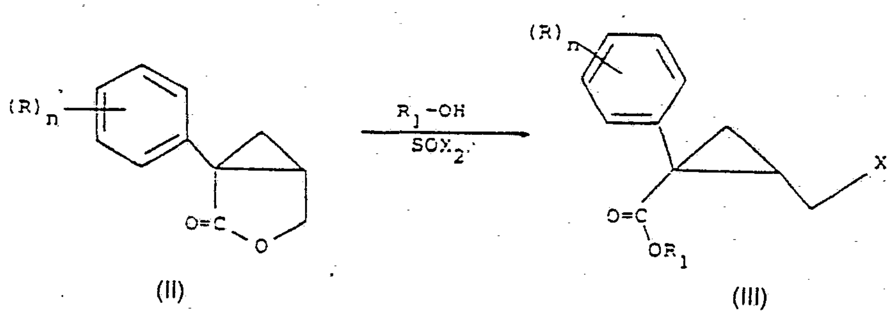


(II) (III)

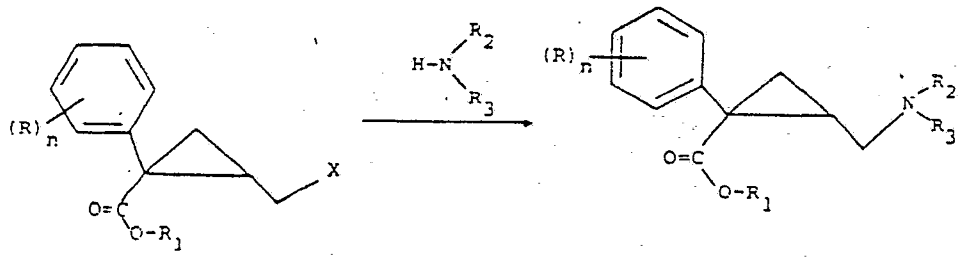

(III) (I)

où

X représente un halogène

R, $R_1$, $R_2$, $R_3$ et n ont la signification donnée précédemment à propos de la formule générale (I).

Les exemples suivants illustrent l'invention sans, bien entendu, en limiter la portée. Dans les formules des composés des exemples suivants, le symbole Et désigne le radical éthyle.

Exemple A (exemple type de préparation)

Préparation du chlorhydrate du phényl-1 éthoxy carbonyl-1 pyrrolidino méthyl-2 cyclopropane (Z)

a) préparation du phényl-1 éthoxy carbonyl-1 chlorométhyl-2 cyclopropane (Z)

Dans 500 $cm^3$ d'éthanol à − 10 °C, on introduit sous agitation 126 $cm^3$ (1,8 mole) de chlorure de thionyle en 2 h 30, puis on additionne 100 g (0,57 mole) de phényl-1 oxo-2 oxa-3 bicyclo (3.1.0) hexane. On laisse revenir à température ambiante en maintenant l'agitation pendant 12 heures.

On évapore le solvant réactionnel et on rectifie sous pression réduite.

On récupère avec un rendement de 95 % le produit de formule

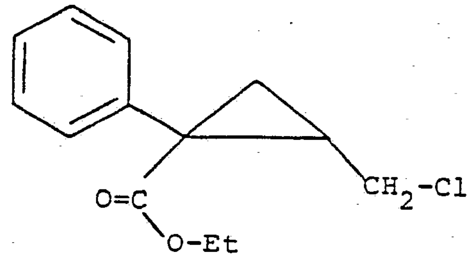

Formule brute : $C_{13}H_{15}ClO_2$
Masse moléculaire : 238,71
Point d'ébullition : 95-98 °C/0,04 mm Hg
Point de fusion : 45-46 °C.

b) chlorhydrate du phényl-1 éthoxy carbonyl-1 pyrrolidino méthyl-2 cyclopropane (Z)

A une solution de (0,03 mole) de phényl-1 éthoxy carbonyl-1 chlorométhyl-2 cyclopropane (Z) dans 60 $cm^3$ de toluène, on ajoute 6,4 g (0,09 mole) de pyrrolidine.

La solution réactionnelle est maintenue au reflux pendant 5 heures. Le solvant est évaporé jusqu'à siccité.

La masse résiduelle est traitée par une solution bicarbonatée, on extrait à l'éther éthylique, puis on lave à l'eau et on sèche sur sulfate de sodium.

La base libre est traitée par une solution éthanolique saturée d'acide chlorhydrique, par addition d'éther le sel précipite et l'on récupère avec un rendement de 85 % le produit de formule

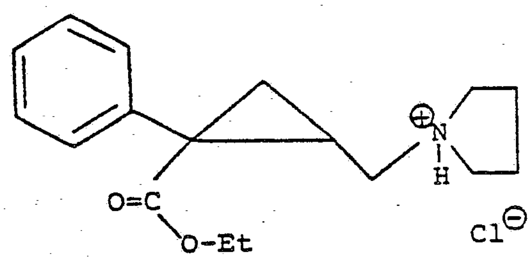

Formule brute : $C_{17}H_{24}ClNO_2$
Masse moléculaire : 309,8
Cristaux : blancs
Point de fusion : 140 °C
Chromatographie sur plaque :
— support : gel de silice 60 F 254 Merck
— solvant : butanol-acide acétique-eau 6/2/2
— révélation : UV et iode
— Rf : 0,49
Solubilité : soluble dans l'eau à 50 %.

Exemple 1

(Chloro-4' phényl)-1 éthoxy carbonyl-1 diméthylaminométhyl-2 cyclopropane (Z), chlorhydrate

D'une façon similaire à celle décrite dans l'exemple A, mais en utilisant la lactone de l'acide p (chlorophényl)-1 hydroxy méthyl-2 cyclopropane carboxylique (Z) et la diméthylamine, on obtient le produit de formule

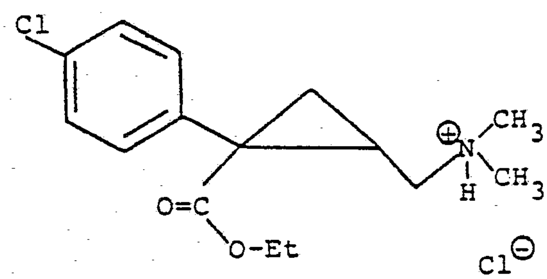

Formule brute : $C_{15}H_{21}Cl_2NO_2$
Masse moléculaire : 318,25
Cristaux : beige-clair
Point de fusion : 132 °C
Chromatographie sur plaque :
— support : gel de silice 60 F 254 Merck
— solvant : chloroforme-méthanol-ammoniaque 84/14/2
— révélation : UV et iode
— Rf : 0,7.

Exemple 2

Chlorhydrate d'(α naphtyl)-1 éthoxy carbonyl-1 diméthylamino méthyl-2 cyclopropane (Z)

D'une façon similaire à celle décrite dans l'exemple A, mais en utilisant la lactone de l'acide α naphtyl hydroxyméthyl-2 cyclopropane carboxylique (Z) et la diméthylamine, on obtient le produit de formule

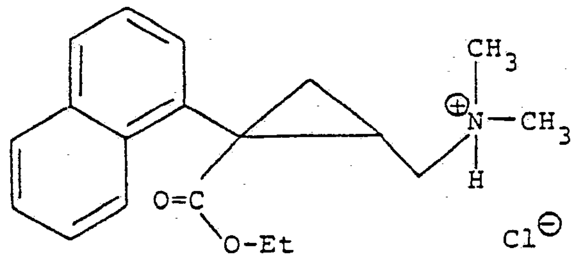

Formule brute : $C_{19}H_{24}ClNO_2$
Masse moléculaire : 333,86
Cristaux : blancs
Point de fusion : 173 °C
Chromatographie sur plaque :
- support : gel de silice 60 F 254 Merck
- solvant : butanol-acide acétique-eau 6/2/2
- révélation : UV et iode
- Rf : 0,34.

## Exemple 3

Chlorhydrate du (sulfamoyl-4' phényl)-1 méthoxy carbonyl-1 diméthylamino méthyl-2 cyclopropane (Z).

D'une façon similaire à celle décrite dans l'exemple A, mais en utilisant le (sulfamoyl-4' phényl)-1 oxo-2 oxa-3 bicyclo (3 : 1 : 0) hexane, l'alcool méthylique et la diméthylamine, on obtient le produit de formule

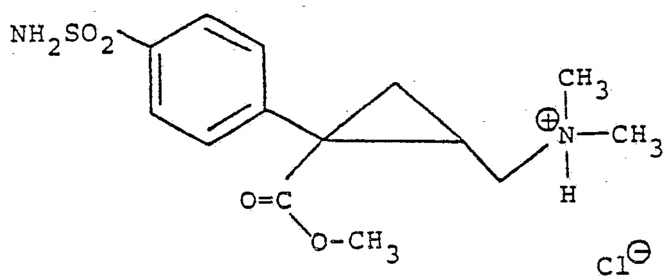

Formule brute : $C_{14}H_{21}ClNO_4S$
Masse moléculaire : 348,8
Cristaux : beige-clair
Point de fusion : 214 °C
Chromatographie sur plaque :
- support : gel de silice 60 F 254 Merck
- solvant : chloroforme-méthanol-eau 65/25/4
- révélation : UV et iode
- Rf : 0,25.

## Exemple 4

Maléate de m-chlorophényl-1 éthoxy carbonyl-1 diméthylamino méthyl-2 cyclopropane (Z)

D'une façon similaire à celle décrite dans l'exemple A, mais en utilisant la lactone de l'acide m (chlorophényl)-1 hydroxy méthyl-2 cyclopropane carboxylique (Z) et la diméthylamine, on obtient le produit de formule

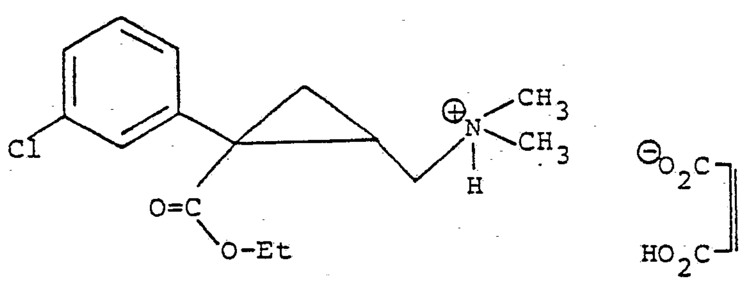

Formule brute : $C_{19}H_{24}ClNO_6$
Masse moléculaire : 397,8
Cristaux : blancs
Point de fusion : 85 °C
Chromatographie sur plaque :
— support : gel de silice 60 F 254 Merck
— solvant : butanol-acide acétique-eau 6/2/2
— révélation : UV et iode
— Rf : 0,42.

Exemple 5

Chlorhydrate du (dichloro-3-4 phényl)-1 éthoxy carbonyl-1 diméthylaminométhyl-2 cyclopropane (Z)

D'une façon similaire à celle décrite dans l'exemple A, mais en utilisant le (dichloro-3'-4' phényl)-1 oxo-2 oxa-3 bicyclo (3 : 1 : 0) hexane et la diméthylamine on obtient le produit de formule

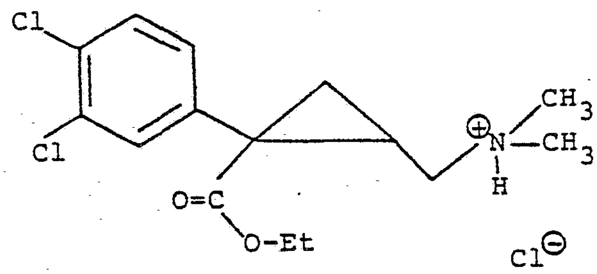

Formule brute : $C_{15}H_{20}Cl_3NO_2$
Masse moléculaire : 352,7
Cristaux : blancs
Point de fusion : 148 °C
Chromatographie sur plaque :
— support : gel de silice 60 F 254 Merck
— solvant : butanol-acide acétique-eau 6/2/2
— révélation : UV et iode
— Rf : 0,37.

Exemple 6

Maléate de (p-aminophényl)-1 méthyloxy carbonyl-1 diméthylamino méthyl-2 cyclopropane (Z)

D'une façon similaire à celle décrite dans l'exemple A, mais en utilisant le (p-aminophényl)-1 oxo-2 oxa-3 bicyclo (3 : 1 : 0) hexane, le méthanol et la diméthylamine on obtient le produit de formule

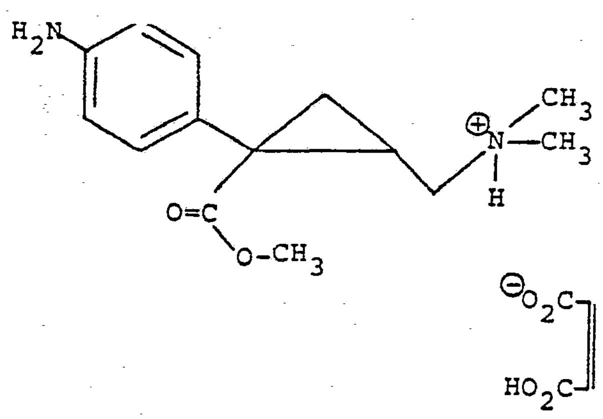

Formule brute : $C_{18}H_{24}N_2O_6$
Masse moléculaire : 364,4
Cristaux : blancs
Point de fusion : 110 °C
Chromatographie sur plaque :

— support : gel de silice 60 F 254 Merck
— solvant : chloroforme-méthanol-ammoniaque 80/18/2
— révélation : UV et iode
— Rf : 0,66.

Exemple 7

Chlorhydrate de (p-nitrophényl)-1 méthoxy carbonyl-1 diméthyl aminométhyl-2 cyclopropane (Z)

D'une façon similaire à celle décrite dans l'exemple A, mais en utilisant le (nitro-4' phényl)-1 oxo-2 oxa-3 bicyclo (3 : 1 : 0) hexane, le méthanol et la diméthylamine, on obtient le produit de formule

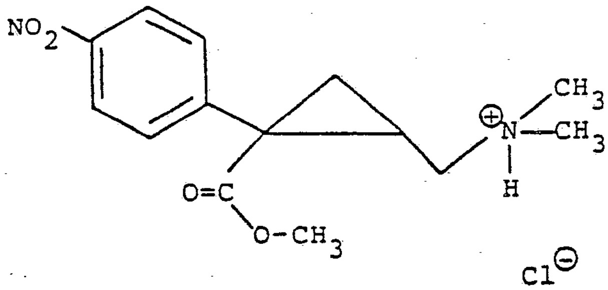

Formule brute : $C_{14}H_{19}ClN_2O_4$
Masse moléculaire : 314,8
Cristaux : blancs
Point de fusion : 210 °C
Chromatographie sur plaque :
— support : gel de silice 60 F 254 Merck
— solvant : chloroforme-méthanol-eau 80/18/2
— révélation : UV et iode
— Rf : 0,64.

Exemple 8

Chlorhydrate de (p-méthoxy phényl)-1 éthoxy carbonyl-1 diméthyl aminométhyl-2 cyclopropane (Z)

D'une façon similaire à celle décrite dans l'exemple A, mais en utilisant le (p-méthoxy phényl)-1 oxo-2 oxa-3 bicyclo (3 : 1 : 0) hexane et la diméthylamine, on obtient le produit de formule

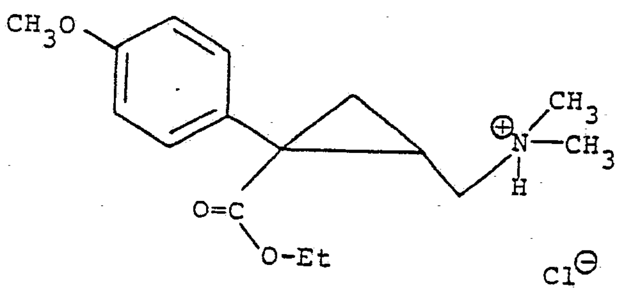

Formule brute : $C_{16}H_{24}ClNO_3$
Masse moléculaire : 313,8
Cristaux : blancs
Point de fusion : 170 °C
Chromatographie sur plaque :
— support : gel de silice 60 F 254 Merck
— solvant : butanol-acide acétique-eau 6/2/2
— révélation : UV et iode
— Rf : 0,49.

Exemple 9

Chlorhydrate de p-toluyl-1(propen-2' yloxy carbonyl)-1 diméthyl amino méthyl-2 cyclopropane (Z)

D'une façon similaire à celle décrite dans l'ensemble A, mais en utilisant le p-toluyl-1 oxo-2 oxa-3 bicyclo (3 : 1 : 0) hexane, l'alcool allylique et la diméthylamine, on obtient le produit de formule

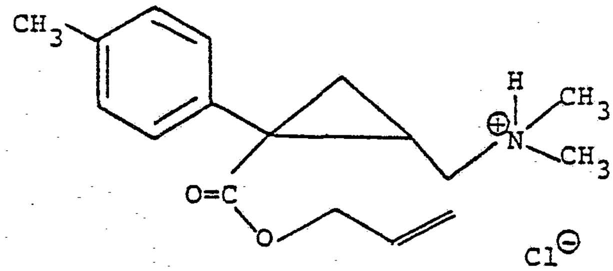

Formule brute : $C_{17}H_{24}ClNO_2$
Masse moléculaire : 309,8
Cristaux : blancs
Point de fusion : 154 °C
Chromatographie sur plaque :
— support : gel de silice 60 F 254 Merck
— solvant : chloroforme-méthanol 85/15
— révélation : UV et iode
— Rf : 0,41

Exemple 10

Chlorhydrate de (dichloro-3'-4' phényl)-1 (propen-2' yloxy carbonyl)-1 diméthylaminométhyl-2 cyclopropane (Z)

D'une façon similaire à celle décrite dans l'exemple A, mais en utilisant le (dichloro-3'-4' phényl)-1 oxo-2 oxa-3 bicyclo (3 : 1 : 0) hexane, l'alcool allylique et la diméthylamine, on obtient le produit de formule

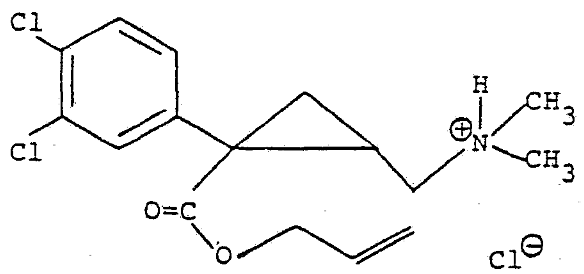

Formule brute : $C_{16}H_{20}Cl_3NO_2$
Masse moléculaire : 364,7
Cristaux : blancs
Point de fusion : 160 °C
Chromatographie sur plaque :
— support : gel de silice 60 F 254 Merck
— solvant : chloroforme-méthanol 15/85
— révélation : UV et iode
— Rf : 0,45.

Exemple 11

Chlorhydrate de parafluorophényl-1 éthoxy carbonyl-1 diméthyl aminométhyl-2 cyclopropane (Z)

D'une façon similaire à celle décrite dans l'exemple A, mais en utilisant le p-fluorophényl-1 oxo-2 oxa-3 bicyclo (3 :1 : 0) hexane et la diméthylamine, on obtient le produit de formule

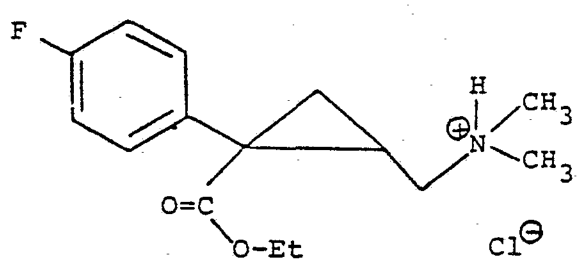

Formule brute : $C_{15}H_{21}$ $FClNO_2$
Masse moléculaire : 301,7
Cristaux : blancs
Point de fusion : 165 °C
Chromatographie sur plaque :
- support : gel de silice 60 F 254 Merck
- solvant : butanol-acide acétique-eau
- révélation : UV et iode
- Rf : 0,36.

Exemple 12

Chlorhydrate de p-chlorophényl-1 éthoxy carbonyl-1 aminométhyl-2 cyclopropane (Z)

D'une façon similaire à celle décrite dans l'exemple A, mais en utilisant le p-chlorophényl-1 oxo-2 oxa-3 bicyclo (3 : 1 : 0) hexane, et l'ammoniaque, on obtient le produit de formule

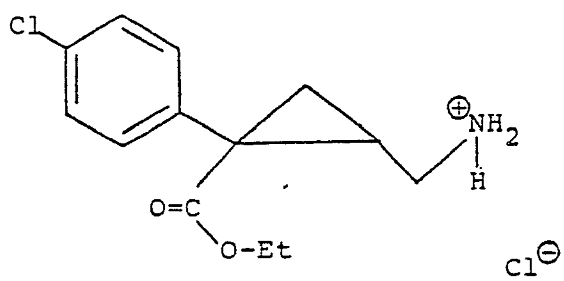

Exemple 13

Chlorhydrate du p-hydroxyphényl-1 éthoxy carbonyl-1 diméthyl aminométhyl-2 cyclopropane (Z)

D'une façon similaire à celle décrite dans l'exemple A, mais en utilisant le p-hydroxyphényl-1 oxo-2 oxa-3 bicyclo (3 : 1 : 0) hexane, et la diméthylamine, on obtient le produit de formule

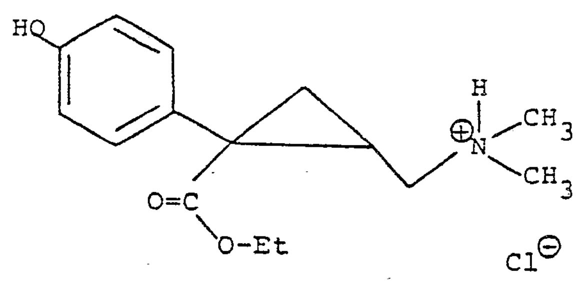

Exemple 14

Chlorhydrate du (triméthoxy-3'-4'-5' phényl)-1 éthoxy carbonyl-1 diméthylaminométhyl-2 cyclopropane (Z)

D'une façon similaire à celle décrite dans l'exemple A, mais en utilisant le (triméthoxy-3'-4'-5' phényl)-1 oxo-2 oxa-3 bicyclo (3 : 1 : 0) hexane et la diméthylamine, on obtient le produit de formule

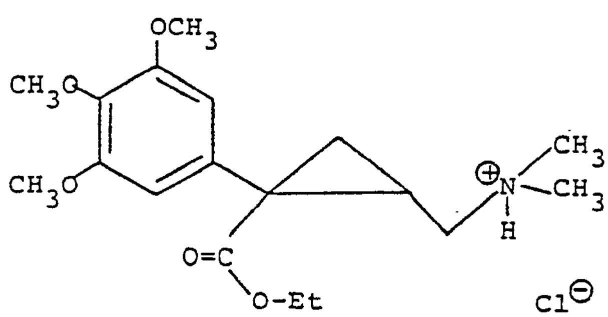

Exemple 15

Chlorhydrate de l'ortho bromophényl-1 éthoxy carbonyl-1 diméthylamino méthyl-2 cyclopropane (Z)

D'une façon similaire à celle décrite dans l'exemple A, mais en utilisant l'ortho bromophényl-1 oxo-2 oxa-3 bicyclo (3 : 1 : 0) hexane et la diméthylamine, on obtient le produit de formule :

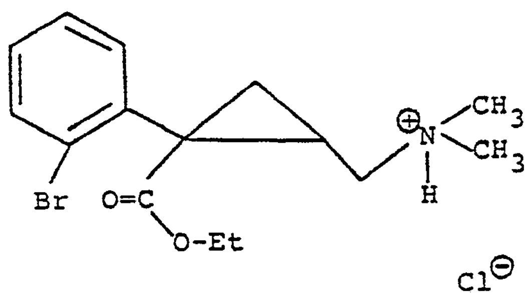

Propriétés pharmacologiques

1) Etude de la toxicité

Les composés les plus intéressants de la présente invention ont été soumis à des contrôles de toxicité.

Les doses létales 50 ont été recherchées par voie orale et intrapéritonéale et calculées selon la méthode de Miller et Tainter (Proc. Soc. Exper. Biol. Chem. 1944, 57, 261).

Les résultats sont rapportés dans le tableau I ci-après.

Tableau 1

| Produits | $DE_{50}$ v.o. | $DE_{50}$ i.p. | writhing-syndrome PBQ-$DE_{50}$ mg/kg (P.O) | plaque chauffante 50 mg/kg (SC) tps réaction (seconde) |
|---|---|---|---|---|
| Exemple 1 | 400 | 120 | 19 | 39 |
| Exemple 4 | 760 | 170 | 30 | 28 |
| Exemple 5 | 550 | 170 | 60 | 26 |
| Exemple 6 | 420 | 100 | 34 | 25 |
| Exemple 7 | 1 000 | 250 | 21 | 20 |
| Exemple 8 | 420 | 110 | 95 | 29 |
| Dextropropoxyphène | | | 30 | 30 |

2) Activité antalgique

a) Writhing-syndrome (P. B. Q.)

Après administration I. P. chez la souris de p-benzoquinone les produits sont testés selon la méthode de R. Okun, S. C. Liddon et L. Lasagna, J. Pharmacol. Exptl. Therap., 1963, 139, 107. Les résultats sont exprimés en $DE_{50}$ dans le tableau I.

b) plaque chauffante

Les produits sont testés chez la souris par voie sous-cutanée (50 mg/kg) selon la méthode de N. B. Eddy et D. Leimbach, J. Pharm. Exptl. Therap., 1953, 107, 385. Les résultats sont exprimés dans le tableau I.

3) Applications thérapeutiques

Compte tenu de leurs propriétés pharmacologiques et de leur faible toxicité, ces composés peuvent

être utilisés en thérapeutique dans le traitement d'algies diverses et plus particulièrement les composés des exemples 1, 4, 6, 7.

Ces composés et leurs sels d'addition avec des acides thérapeutiquement compatibles peuvent être utilisés comme médicaments par exemple sous forme de préparations pharmaceutiques adaptées facilitant la biodisponibilité.

Ces préparations peuvent se présenter sous forme solide par exemple de comprimés, dragées, capsules, etc. ou sous forme liquide, par exemple de solutions, suspensions ou émulsions.

Les préparations pharmaceutiques sous une forme appropriée à l'injection sont soumises à des opérations pharmaceutiques classiques telles que stérilisation et/ou peuvent contenir des adjuvants par exemple des agents conservateurs, stabilisants, de mouillage ou d'émulsification, des composés tampons, etc.

Les dosages auxquels les composés actifs et leurs sels d'addition avec des acides thérapeutiquement compatibles peuvent être administrés, peuvent varier dans des proportions importantes selon l'état du patient. Un dosage quotidien d'environ 0,1 mg à 1 mg/kg de poids corporel est toutefois préféré.

Les compositions pharmaceutiques selon l'invention peuvent être utilisées en médecine humaine ou vétérinaire, par exemple dans le traitement des phénomènes douloureux notamment en cancérologie, traumatologie, rhumatologie, neurologie et chirurgie.

D'autres principes actifs peuvent être associés aux composés de formule générale I selon l'invention pour compléter ou renforcer leurs actions thérapeutiques au sein d'une même composition pharmaceutique.

Exemples de préparations pharmaceutiques

A. Comprimés à effet prolongé

| | |
|---|---|
| Chlorhydrate de (chloro-4' phényl)-1 éthoxy carbonyl-1 diméthylaminométhyl-2 cyclopropane (Z) | 50 mg |
| Excipient retard Q. S. P. | 1 comprimé |

B. Suppositoire

| | |
|---|---|
| Chlorhydrate de (chloro-4' phényl)-1 éthoxy carbonyl-1 diméthylaminométhyl-2 cyclopropane (Z) | 25 mg |
| Paracétamol | 300 mg |
| Excipient Q. S. P. | 1 suppositoire adulte |

**Revendications** (pour les Etats contractants : BE, CH, DE, GB, IT, LI, LU, NL, SE)

1. Dérivés d'aryl-1 aminométhyl-2 cyclopropanes carboxylates (Z) répondant à la formule générale 1

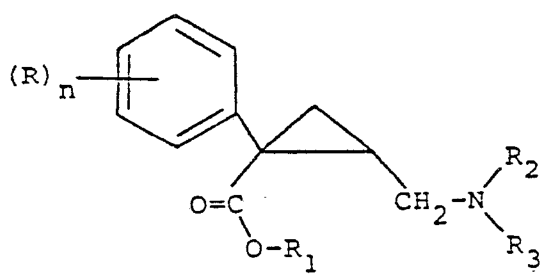

(I)

dans laquelle

R représente un atome d'halogène, un groupe alcoyle inférieur en $C_1$ à $C_4$, alcoxy inférieur en $C_1$ à $C_4$, nitro, amino, sulfamoyle ou hydroxy ;

n représente les valeurs 1, 2 ou 3 ;

$(R)_n$ peut également former avec le cycle benzénique le noyau naphtyle :

$R_1$ représente un groupe alcoyle ou alcényle linéaires ou ramifiés en $C_1$ à $C_5$, aryle ou benzyle ;

$R_2$ et $R_3$ représentent un atome d'hydrogène, un groupe alcoyle, alcényle, alcynyle, hydroxyalcoyle, alcoxyalcoyle, carboxyalcoyle ou dialcoylaminoalcoyle linéaires ou ramifiés en $C_1$ à $C_5$, aryle, arylalcoyle ou cycloalcoyle ;

$R_2$ et $R_3$ pouvant également former avec l'atome d'azote voisin un hétérocycle à 5 ou 6 chaînons, ainsi que leurs sels avec des acides minéraux ou organiques thérapeutiquement acceptables.

2. Le (chloro-4' phényl)-1 éthoxy carbonyl-1 diméthylaminométhyl-2 cyclopropane (Z), chlorhydrate selon la revendication 1.

3. Le maléate de m-chlorophényl-1 éthoxy carbonyl-1 diméthylamino méthyl-2 cyclopropane (Z) selon la revendication 1.

4. Le maléate de (p-aminophényl)-1 méthyloxy carbonyl-1 diméthylaminométhyl-2 cyclopropane (Z) selon la revendication 1.

5. Le chlorhydrate de (p-nitrophényl)-1 méthoxy carbonyl-1 diméthylaminométhyl-2 cyclopropane (Z) selon la revendication 1.

6. Le chlorhydrate de (p-méthoxy phényl)-1 éthoxy carbonyl-1 diméthylaminométhyl-2 cyclopropane (Z) selon la revendication 1.

7. Procédé de préparation des composés de formule générale I selon l'une des revendications 1 à 6, caractérisé en ce que l'on traite la lactone de formule générale II

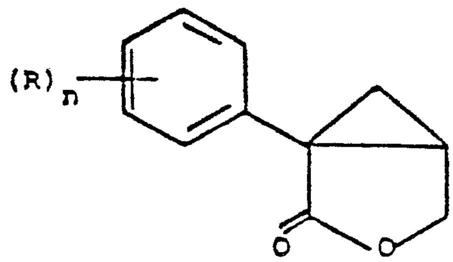

(II)

par un alcool ou un phénol de formule $R_1$-OH, en présence d'un halogénure de thionyle de formule $SOX_2$, pour obtenir les dérivés intermédiaires de synthèse de formule III

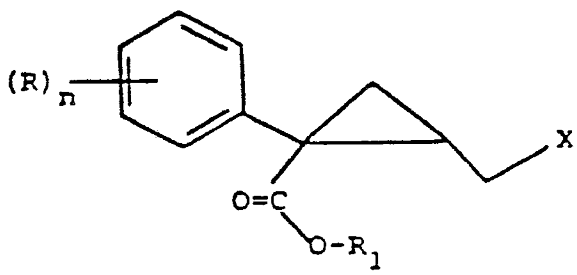

(III)

ces dérivés de formule III étant ensuite traités par une amine de formule générale IV

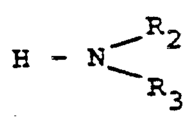

(IV)

pour donner les composés de formule générale I, X représentant un atome d'halogène et R, $R_1$, $R_2$, $R_3$ et n ont la signification donnée à propos de la formule générale I.

8. A titre de médicaments nouveaux les composés selon l'une des revendications 1 à 6.

9. Compositions pharmaceutiques caractérisées en qu'elles contiennent comme principe actif au moins un composé selon l'une des revendications 1 à 6.

10. Compositions pharmaceutiques selon la revendication 9, caractérisées en ce que le paracétamol est associé aux composés de formule générale I pour compléter ou renforcer leurs actions thérapeutiques.

**Revendications** (pour l'Etat contractant AT)

1. Procédé de préparation des dérivés d'aryl-1 aminométhyl-2 cyclopropanes carboxylates (Z) répondant à la formule générale I

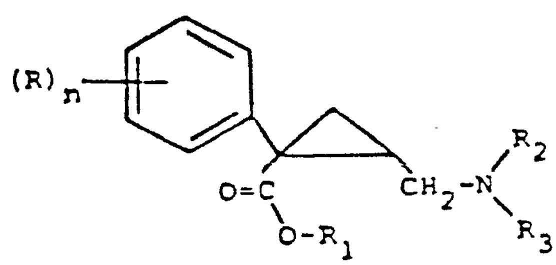

(I)

dans laquelle

R représente un atome d'halogène, un groupe alcoyle inférieur en $C_1$ à $C_4$, alcoxy inférieur en $C_1$ à $C_4$, nitro, amino, sulfamoyle ou hydroxy ;

n représente les valeurs 1, 2 ou 3 ;

$(R)_n$ peut également former avec le cycle benzénique le noyau naphtyle ;

$R_1$ représente un groupe alcoyle ou alcényle linéaires ou ramifiés en $C_1$ à $C_5$, aryle ou benzyle ;

$R_2$ et $R_3$ représentent un atome d'hydrogène, un groupe alcoyle, alcényle, alcynyle, hydroxyalcoyle, alcoxyalcoyle, carboxyalcoyle ou dialcoylaminoalcoyle linéaires ou ramifiés en $C_1$ à $C_5$, aryle, arylalcoyle ou cycloalcoyle ;

$R_2$ et $R_3$ pouvant également former avec l'atome d'azote voisin un hétérocycle à 5 ou 6 chaînons, ainsi que leurs sels avec des acides minéraux ou organiques thérapeutiquement acceptables, caractérisé en ce que l'on traite la lactone de formule générale II

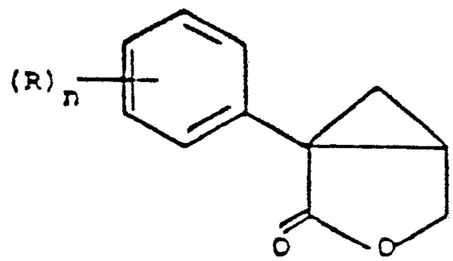

(II)

par un alcool ou un phénol de formule $R_1$-OH, en présence d'un halogénure de thionyle de formule $SOX_2$, pour obtenir les dérivés intermédiaires de synthèse de formule III

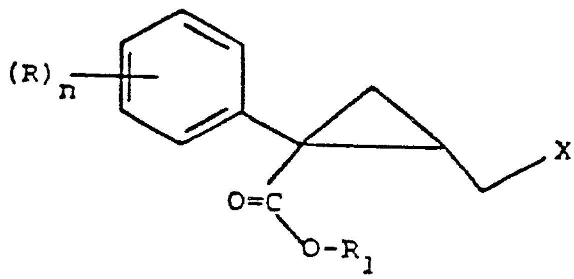

(III)

ces dérivés de formule III étant ensuite traités par une amine de formule générale IV

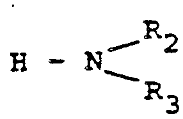

(IV)

pour donner les composés de formule générale I, X représentant un atome d'halogène et R, $R_1$, $R_2$, $R_3$ et n ont la signification donnée à propos de la formule générale I.

2. Procédé selon la revendication 1, caractérisé en ce que l'on prépare le (chloro-4' phényl)-1 éthoxy carbonyl-1 diméthylaminométhyl-2 cyclopropane (Z), chlorhydrate.

3. Procédé selon la revendication 1, caractérisé en ce que l'on prépare le maléate de m-chlorophényl-1 éthoxy carbonyl-1 diméthylamino méthyl-2 cyclopropane (Z).

4. Procédé selon la revendication 1, caractérisé en ce que l'on prépare le maléate de (p-aminophényl)-1 méthyloxy carbonyl-1 diméthylaminométhyl-2 cyclopropane (Z).

5. Procédé selon la revendication 1, caractérisé en ce que l'on prépare le chlorhydrate de (p-nitrophényl)-1 méthoxy carbonyl-1 diméthylaminométhyl-2 cyclopropane (Z).

6. Procédé selon la revendication 1, caractérisé en ce que l'on prépare le chlorhydrate de (p-méthoxy phényl)-1 éthoxy carbonyl-1 diméthyl aminométhyl-2 cyclopropane (Z).

**Claims** (for the Contracting States : BE, CH, DE, GB, IT, LI, LU, NL, SE)

1. Derivatives of (Z)-1-aryl-2-(aminomethyl) cyclopropanecarboxylates corresponding to the general formula I

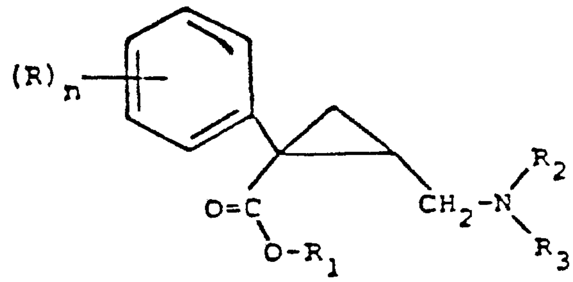

(I)

in which

R denotes a halogen atom, a lower $C_1$ to $C_4$ alkyl group, a lower $C_1$ to $C_4$ alkoxy group, or a nitro, amino, sulphamoyl or hydroxy group ;

n denotes the values 1, 2 or 3 ;

$(R)_n$ can also form a naphthalene nucleus with the benzene ring ;

$R_1$ denotes a $C_1$ to $C_5$ linear or branched alkyl or alkenyl group, or an aryl or benzyl group ;

$R_2$ and $R_3$ denote a hydrogen atom, or a $C_1$ to $C_5$ linear or branched alkyl, alkenyl, alkynyl, hydroxyalkyl, alkoxyalkyl, carboxyalkyl or dialkylaminoalkyl group, or an aryl, arylalkyl or cycloalkyl group ;

$R_2$ and $R_3$ can also form a 5- or 6-membered heterocycle with the neighbouring nitrogen atom, as well as their salts with therapeutically acceptable inorganic or organic acids.

2. (Z)-1-(4'-Chlorophenyl)-1-ethoxycarbonyl-2-(dimethylaminomethyl) cyclopropane hydrochloride, according to Claim 1.

3. (Z)-1-(m-Chlorophenyl)-1-ethoxycarbonyl-2-(dimethylaminomethyl) cyclopropane maleate, according to Claim 1.

4. (Z)-1-(p-Aminophenyl)-1-methoxycarbonyl-2-(dimethylaminomethyl) cyclopropane maleate, according to Claim 1.

5. (Z)-1-(p-Nitrophenyl)-1-methoxycarbonyl-2-(dimethylaminomethyl) cyclopropane hydrochloride, according to Claim 1.

6. (Z)-1-(p-Methoxyphenyl)-1-ethoxycarbonyl-2-(dimethylaminomethyl) cyclopropane hydrochloride, according to Claim 1.

7. Process for preparing the compounds of general formula I according to one of claims 1 to 6, characterised in that the lactone of general formula II

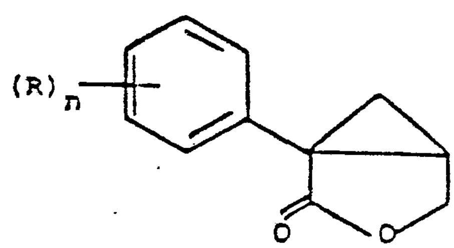

(II)

is treated with an alcohol or phenol of formula $R_1$-OH in the presence of a thionylhalide of formula $SOX_2$, to produce the intermediate synthesis derivatives of formula III

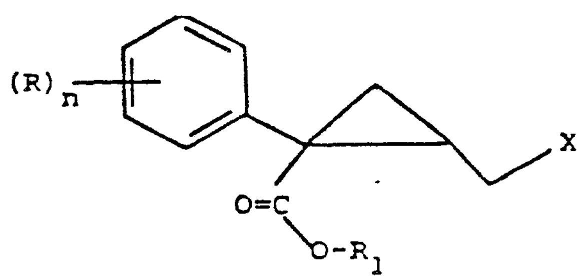

(III)

these derivatives of formula III then being treated with an amine of general formula IV

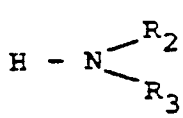

(IV)

to give the compounds of general formula I, X denoting a halogen atom and R, $R_1$, $R_2$, $R_3$ and n having the significance given in relation to the general formula I.

8. By way of new drugs, the compounds according to one of Claims 1 to 6.

9. Pharmaceutical compositions, characterised in that they contain, as active principle, at least one compound according to one of Claims 1 to 6.

10. Pharmaceutical compositions according to Claim 9, characterised in that paracetamol is mixed with the compounds of general formula I to complete or reinforce their therapeutic actions.

**Claims** (for the Contracting State AT)

1. Process for preparing derivatives of (Z)-1-aryl-2-(aminomethyl) cyclopropanecarboxylates corresponding to the general formula I

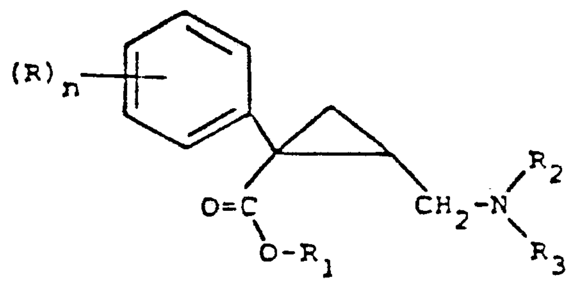

(I)

in which

R denotes a halogen atom, a lower $C_1$ to $C_4$ alkyl group, a lower $C_1$ to $C_4$ alkoxy group, or a nitro, amino, sulphamoyl or hydroxy group ;

n denotes the values 1, 2 or 3 ;

$(R)_n$ can also form a naphthalene nucleus with the benzene ring ;

$R_1$ denotes a $C_1$ to $C_5$ linear or branched alkyl or alkenyl group, or an aryl or benzyl group ;

$R_2$ and $R_3$ denote a hydrogen atom, or a $C_1$ to $C_5$ linear or branched alkyl, alkenyl, alkynyl, hydroxyalkyl, alkoxyalkyl, carboxyalkyl or dialkylaminoalkyl group, or an aryl, arylalkyl or cycloalkyl group ;

$R_2$ and $R_3$ can also form a 5- or 6-membered heterocycle with the neighbouring nitrogen atom, as well as their salts with therapeutically acceptable inorganic or organic acids, characterised in that the lactone of general formula II

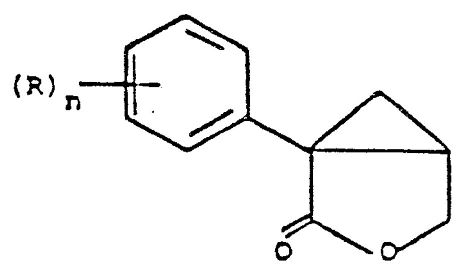

(II)

is treated with an alcohol or phenol of formula $R_1$-OH in the presence of a thionyl halide of formula $SOX_2$, to produce the intermediate synthesis derivatives of formula III

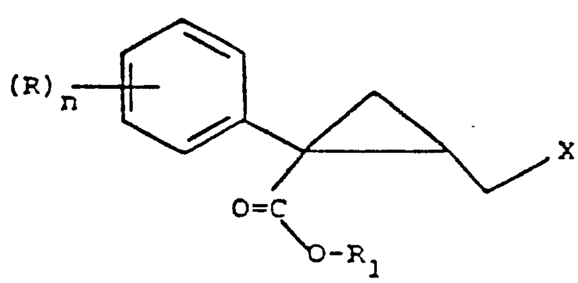

(III)

these derivatives of formula III then being treated with an amine of general formula IV

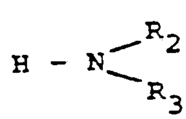

(IV)

the give the compounds of general formula I, X denoting a halogen atom and R, $R_1$, $R_2$, $R_3$ and n having the significance given in relation to the general formula I.

2. Process according to Claim 1, characterised in that (Z)-1-(4′-chlorophenyl)-1-ethoxycarbonyl-2-(dimethylaminomethyl) cyclopropane hydrochloride is prepared.

3. Process according to Claim 1, characterised in that (Z)-1-(m-chlorophenyl)-1-ethoxycarbonyl-2-(dimethylaminomethyl) cyclopropane maleate is prepared.

4. Process according to Claim 1, characterised in that (Z)-1-(p-aminophenyl)-1-methoxycarbonyl-2-(dimethylaminomethyl) cyclopropane maleate is prepared.

5. Process according to Claim 1, characterised in that (Z)-1-(p-nitrophenyl)-1-methoxycarbonyl-2-(dimethylaminomethyl) cyclopropane hydrochloride is prepared.

6. Process according to Claim 1, characterised in that (Z)-1-(p-methoxyphenyl)-1-ethoxycarbonyl-2-(dimethylaminomethyl) cyclopropane hydrochloride is prepared.

**Patentansprüche** (für die Vertragsstaaten : BE, CH, DE, GB, IT, LI, LU, NL, SE)

1. 1-Aryl-2-aminomethylcyclopropancarboxylat (Z)-derivate der allgemeinen Formel

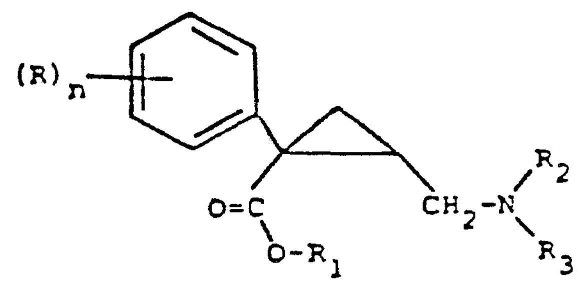

(I)

worin

R ein Halogenatom, eine nied. Alkylgruppe mit 1 bis 4 C-Atomen, eine nied. Alkoxygruppe mit 1 bis 4 C-Atomen, eine Nitro-, Amino-, Sulfamoyl- oder Hydroxygruppe bedeutet ;

n die Werte 1, 2 oder 3 darstellt ;

$(R)_n$ auch mit dem Benzolring das Naphthylringsystem bilden kann ;

$R_1$ eine gerade oder verzweigte Alkyl- oder Alkenylgruppe mit 1 bis 5 C-Atomen, Aryl oder Benzyl sein kann ;

$R_2$ und $R_3$ ein Wasserstoffatom, eine gerade oder verzweigte Alkyl-, Alkenyl-, Alkinyl-, Hydroxyalkyl-, Alkoxyalkyl-, Carboxyalkyl- oder Dialkylaminoalkylgruppe mit 1 bis 5 C-Atomen, oder eine Aryl-, Arylalkyl- oder Cycloalkylgruppe darstellen,

$R_2$ und $R_3$ aber auch zusammen mit dem benachbarten Stickstoffatom einen Heterocyclus mit 5 oder 6 Gliedern bilden können, sowie deren Salze mit Mineralsäuren oder therapeutisch annehmbaren organischen Säuren.

2. Das 1-(4′-Chlorphenyl)-1-äthoxycarbonyl-2-dimethylaminomethylcyclopropan (Z)-chlorhydrat gemäß Anspruch 1.

3. Das Maleat von 1-m-Chlorphenyl-1-äthoxycarbonyl-2-dimethylaminomethyl-cyclopropan (Z) gemäß Anspruch 1.

4. Das Maleat von 1-(p-Aminophenyl)-1-methyloxycarbonyl-2-dimethylaminomethyl-cyclopropan (Z) gemäß Anspruch 1.

5. Das Chlorhydrat von 1-(p-Nitrophenyl)-1-methoxycarbonyl-2-dimethylaminomethyl-cyclopropan (Z) gemäß Anspruch 1.

6. Das Chlorhydrat von 1-(p-Methoxyphenyl)-1-äthoxycarbonyl-2-dimethylaminomethyl-cyclopropan (Z) gemäß Anspruch 1.

7. Verfahren zur Herstellung der Verbindungen der allgemeinen Formel (I) gemäß einem der Ansprüche 1 bis 6, dadurch gekennzeichnet, daß man das Lacton der allgemeinen Formel (II)

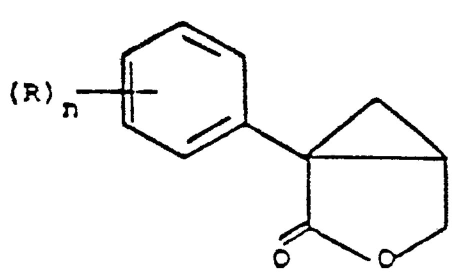

(II)

mit einem Alkohol oder einem Phenol der Formel $R_1$-OH in Anwesenheit eines Thionylhalogenids der Formel $SOX_2$ zur Erzielung der Synthesezwischenprodukte der Formel (III)

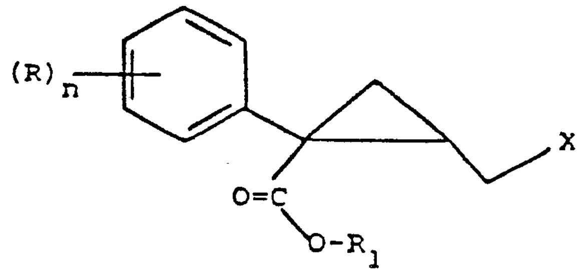

(III)

behandelt, welche Derivate der Formel (III) anschließend mit einem Amin der Allgemeinen Formel (IV)

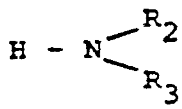

(IV)

behandelt werden, wobei die Verbindungen der allgemeinen Formel (I) erhalten werden, worin X ein Halogenatom bedeutet und R, $R_1$, $R_2$, $R_3$ und n die bezüglich der allgemeinen Formel (I) angegebenene Bedeutung haben.

8. Die Verbindungen gemäß einem der Ansprüch 1 bis 6 als neue Medikamente.

9. Pharmazeutische Zusammensetzungen, dadurch gekennzeichnet, daß sie als Wirkstoff mindestens eine Verbindung gemäß einem der Ansprüche 1 bis 6 enthalten.

10. Pharmazeutische Zusammensetzungen gemäß Anspruch 9, dadurch gekennzeichnet, daß das Paracetamol mit Verbindungen der allgemeinen Formel (I) kombiniert ist, um deren therapeutische Wirkungen zu vervollständigen oder zu verstärken.

**Patentansprüche** (für den Vertragsstaat AT)

1. Verfahren zur Herstellung von 1-Aryl-2-aminomethylcyclopropancarboxylat (Z)-derivaten der allgemeinen Formel (I)

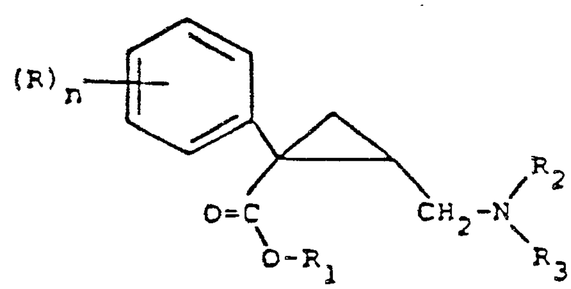

(I)

worin

R ein Halogenatom, eine nied. Alkylgruppe mit 1 bis 4 C-Atomen, eine nied. Alkoxygruppe mit 1 bis 4 C-Atomen, eine Nitro-, Amino-, Sulfamoyl- oder Hydroxygruppe bedeutet ;

n die Werte 1, 2 oder 3 darstellt ;

$(R)_n$ auch mit dem Benzolring das Naphthylringsystem bilden kann ;

$R_1$ eine gerade oder verzweigte Alkyl- oder Alkenylgruppe mit 1 bis 5 C-Atomen, Aryl oder Benzyl sein kann ;

$R_2$ und $R_3$ ein Wasserstoffatom, eine gerade oder verzweigte Alkyl-, Alkenyl-, Alkinyl-, Hydroxyalkyl-, Alkoxyalkyl-, Carboxyalkyl- oder Dialkylaminoalkylgruppe mit 1 bis 5 C-Atomen, oder eine Aryl-, Arylalkyl- oder Cycloalkylgruppe darstellen,

$R_2$ und $R_3$ aber auch zusammen mit dem benachbarten Stickstoffatom einen Heterocyclus mit 5 oder 6 Gliedern bilden können, sowie deren Salzen mit Mineralsäuren oder therapeutisch annehmbaren organischen Säuren, dadurch gekennzeichnet, daß man das Lacton der allgemeinen Formel (II)

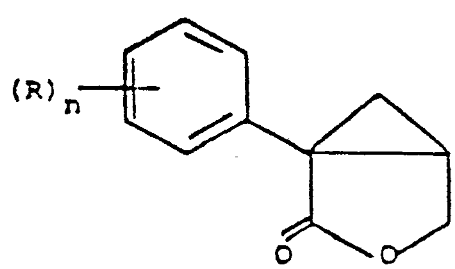

(II)

mit einem Alkohol oder einem Phenol der Formel $R_1$-OH in Anwesenheit eines Thionylhalogenids der Formel $SOX_2$ zur Erzielung der Synthesezwischenprodukte der Formel (III)

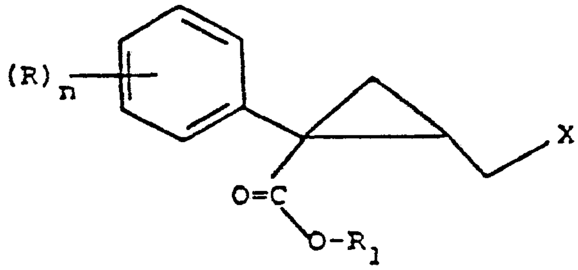

(III)

behandelt, welche Derivate der Formel (III) anschließend mit einem Amin der allgemeinen Formel (IV)

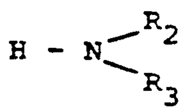

(IV)

behandelt werden, wobei die Verbindung der allgemeinen Formel (I) erhalten werden, worin X ein Halogenatom bedeutet und R, $R_1$, $R_2$, $R_3$ und n die bezüglich der allgemeinen Formel (I) angegebene Bedeutung haben.

2. Verfahren gemäß Anspruch 1, dadurch gekennzeichnet, daß man das Chlorhydrat von 1-(4'-Chlorphenyl)-1-äthoxycarbonyl-2-dimethylaminomethyl-cyclopropan (Z) herstellt.

3. Verfahren gemäß Anspruch 1, dadurch gekennzeichnet, daß man das Maleat von 1-m-Chlorphenyl-1-äthoxycarbonyl-2-dimethylaminomethyl-cyclopropan (Z) herstellt.

4. Verfahren gemäß Anspruch 1, dadurch gekennzeichnet, daß man das Maleat von 1-(p-Aminophenyl)-1-methyloxycarbonyl-2-dimethylaminomethyl-cyclopropan (Z) herstellt.

5. Verfahren gemäß Anspruch 1, dadurch gekennzeichnet, daß man das Chlorhydrat von 1-(p-Nitrophenyl)-1-methoxycarbonyl-2-dimethylaminomethyl-cyclopropan (Z) herstellt.

6. Verfahren gemäß Anspruch 1, dadurch gekennzeichnet, daß man das Chlorhydrat von 1-(p-Methoxyphenyl)-1-äthoxycarbonyl-2-dimethylaminomethyl-cyclopropan (Z) herstellt.